# EUROPÄISCHE PATENTSCHRIFT

(11) **EP 4 245 237 B1**
(45) Veröffentlichungstag und Bekanntmachung des Hinweises auf die Patenterteilung: **25.11.2026**
(21) Anmeldenummer: 22162783.9
(22) Anmeldetag: 17.03.2022
(51) Int. Cl.: A61B 18/14, A61B 18/16, A61B 18/12, A61B 18/00, A61B 17/00

(54) **ELEKTROCHIRURGIESYSTEM UND VERFAHREN ZUM PRÜFEN DER ELEKTRISCHEN VERBINDUNG ZWISCHEN EINER NEUTRALELEKTRODE UND EINEM PATIENTEN**
ELECTROSURGICAL SYSTEM AND METHOD FOR TESTING THE ELECTRICAL CONNECTION BETWEEN A NEUTRAL ELECTRODE AND A PATIENT
SYSTÈME D'ÉLECTROCHIRURGIE ET PROCÉDÉ DE VÉRIFICATION DE LA CONNEXION ÉLECTRIQUE ENTRE UNE ÉLECTRODE NEUTRE ET UN PATIENT

(43) Veröffentlichungstag der Anmeldung: 20.09.2023
(73) Patentinhaber: Erbe Elektromedizin GmbH, 72072 Tübingen (DE)
(72) Erfinder: MAIER,Philipp, 72074 Tübingen (DE)
(74) Vertreter: Rüger Abel IP Legal Solutions GmbH

(56) Entgegenhaltungen:
- EP-A1- 2 537 479
- DE-A1- 102018 114 482
- US-A- 6 007 532
- US-A1- 2012 232 548
- US-A1- 2015 282 725
- VALENTINUZZI M E ET AL: "BIOELECTRICAL IMPEDANCE TECHNIQUES IN MEDICINE PART II: MONITORING OF PHYSIOLOGICAL EVENTS BY IMPEDANCE", CRITICAL REVIEWS IN BIOMEDICAL ENGINEERING, CRC PRESS, BOCA RATON, FL, US, vol. 24, no. 4-06, 1 January 1996 (1996-01-01), pages 353 - 466, XP001058276, ISSN: 0278-940X

## Beschreibung

Die Erfindung betrifft ein Elektrochirurgiesystem und ein Verfahren zum Prüfen der elektrischen Verbindung zwischen einer Neutralelektrode und einem Patienten. Das Elektrochirurgiesystem kann insbesondere zur Durchführung des Verfahrens eingerichtet sein. Bei dem Elektrochirurgiesystem handelt es sich insbesondere um ein System mit einem monopolaren Instrument und einer Neutralelektrode, die an ein Versorgungsgerät anschließbar oder angeschlossen sind.

Bei elektrochirurgischen Instrumenten muss während der Behandlung eines Patienten der Stromkreis vom Versorgungsgerät zur Arbeitselektrode des Instruments und von dort zurück zum Versorgungsgerät geschlossen sein. Zu diesem Zweck ist bei einem monopolaren Instrument eine weitere Elektrode, die als Neutralelektrode bezeichnet wird, am Patienten angebracht. Der Strom kann somit vom Versorgungsgerät über die Arbeitselektrode, den Patienten und die Neutralelektrode zurück zum Versorgungsgerät fließen.

Die Stromdichte an der Neutralelektrode darf nicht zu groß werden, um endogene Verbrennungen während des Betriebs des Elektrochirurgiesystems zu vermeiden. Aus diesem Grund ist es erforderlich, dass die verfügbare leitfähige Kontaktfläche der Neutralelektrode mit einem ausreichend großen Flächenanteil in niederohmiger elektrischer Verbindung zum Patienten angebracht ist. Ein zu geringer elektrisch mit dem Patienten verbundener Flächenanteil der Kontaktfläche kann die Stromdichten ansteigen lassen und somit endogene Verbrennungen im Gewebe benachbart zur Neutralelektrode verursachen. Dabei ist nicht nur auf das ursprünglich korrekte Anbringen der Neutralelektrode am Patienten zu achten, sondern die korrekte Anlage der Neutralelektrode am Patienten muss auch während der gesamten Verwendung des Elektrochirurgiesystems aufrechterhalten werden.

Aus DE 10 2018 114 482 A1 ist eine elektrochirurgische Vorrichtung mit einer Steuereinrichtung, einer Aktivelektrode an einem Instrument sowie einer Neutralelektrode am Patienten bekannt. Durch Messen der Impedanz der Neutralelektrode können unterschiedliche Neutralelektroden voneinander unterschieden werden. Hierfür wird die Impedanz bei niedrigen Frequenzen gemessen, um geteilte von nicht geteilten Elektroden unterscheiden zu können, da der Polarisationseffekt nur bei geteilten Elektroden auftritt. Bei nicht geteilten Elektroden fließt kein Strom über das Gewebe, so dass auch keine Polarisationseffekte entstehen.

US 2012/0232548 A1 betrifft ein elektrochirurgisches System zur Überwachung der Kontaktqualität zwischen der Neutralelektrode und dem Patienten. Das Überwachungssystem misst eine Spannung, einen Strom und die Phase in Bezug auf eine Frequenz einer Prüfwellenform. Die Frequenz der Prüfwellenform wird variiert.

US 6 007 532 A betrifft ebenfalls die Kontaktüberwachung zwischen einer Neutralelektrode und dem Patienten. Eine Impedanz wird gemessen und geprüft, ob der Messwert in einem vorgegebenen Bereich liegt.

Impedanzauswertungen bei medizinischen Einrichtungen sind außerdem aus VALENTINUZZI M E ET AL: "BIOELECTRICAL IMPEDANCE TECHNIQUES IN MEDICINE PART II: MONITORING OF PHYSIOLOGICAL EVENTS BY IMPEDANCE", CRITICAL REVIEWS IN BIOMEDICAL ENGINEERING, CRC PRESS, BOCA RATON, Fl, US, Bd. 24, Nr. 4-06, 1. Januar 1996 (1996-01-01), Seiten 353-466, XP001058276, ISSN: 0278-940X und US 2015/282725 A1 bekannt.

EP 2 537 479 A1 beschreibt die Steuerung eines medizinischen Geräts in Abhängigkeit von der Neutralelektrodenimpedanz. Die Impedanz wird mit einem vorgegebenen Schwellenwert verglichen und der Betrieb des Elektrochirurgiesystems bzw. des Behandlungsinstruments wird nur dann freigegeben, wenn die Impedanz der Neutralelektrode in einem vorgegebenen Bereich liegt, z.B. maximal 140 Ohm beträgt.

Eine solche Impedanzmessung mit Schwellwertvergleich ist unzureichend. Ein zu kleiner am Patienten anliegender Flächenanteil der Kontaktfläche der Neutralelektrode kann nicht in allen Fällen über eine derartige Impedanzmessung erkannt werden.

Der Erfindung liegt die Erkenntnis zugrunde, dass die Impedanzänderung der Neutralelektrode abhängig davon ist, an welcher Stelle der Neutralelektrode die vom Patienten abgelösten Flächenanteile der Kontaktfläche sind und an welcher Stelle die noch elektrisch leitend mit dem Patienten verbundenen Flächenanteile der Kontaktfläche sind. Somit besteht bei dem bisher bekannten Verfahren (EP 2 537 479 A1) die Gefahr, dass nicht alle Fälle eines unzureichenden elektrischen Kontakts zwischen der Neutralelektrode und dem Patienten erkannt werden können.

Es kann daher als Aufgabe der vorliegenden Erfindung angesehen werden, ein Elektrochirurgiesystem und ein Verfahren zum Prüfen der elektrischen Verbindung zwischen einer Neutralelektrode und einem Patienten zu schaffen, bei dem eine verbesserte Prüfung der elektrisch leitfähigen Verbindung zwischen der Neutralelektrode und dem Patienten möglich ist.

Diese Aufgabe wird durch ein Elektrochirurgiesystem mit den Merkmalen des Patentanspruches 1 sowie ein Verfahren mit den Merkmalen des Patentanspruches 14 gelöst.

Das erfindungsgemäße Elektrochirurgiesystem weist ein Versorgungsgerät mit einem Neutralanschluss auf, an den eine Neutralelektrode angeschlossen ist. Die Neutralelektrode ist dazu eingerichtet, elektrisch leitend mit einem Patienten verbunden zu werden. Hierzu kann die Neutralelektrode beispielsweise auf die Haut des Patienten aufgeklebt oder anderweitig befestigt werden.

An dem Neutralanschluss für die Neutralelektrode kann das Versorgungsgerät ein sich insbesondere periodisch änderndes Messsignal anlegen. Das Messsignal kann ein Wechselspannungsmesssignal oder ein Wechselstrommesssignal sein. Das Wechselspannungsmesssignal verursacht einen Messstrom, der durch die Neutralelektrode fließt. Das Wechselstrommesssignal verursacht eine Messspannung, die an der Neutralelektrode anliegt. Der Messstrom oder die Messspannung kann am Neutralanschluss des Versorgungsgeräts gemessen und basierend auf dem Wechselspannungsmesssignal und dem Messstrom oder dem Wechselstrommesssignal und der Messspannung kann ein Impedanzistwert für die Neutralelektrode ermittelt werden.

In einer bevorzugten Ausführung wird ein Wechselspannungsmesssignal in Form einer Spannungsquelle statt eines Wechselstrommesssignals in Form einer Stromquelle zum Messen der Impedanz benutzt, um keine nichtlinearen, spannungsabhängigen Impedanzanteile zu messen.

Erfindungsgemäß wird der Impedanzistwert nicht nur bei einer einzigen Messfrequenz, sondern für mehrere verschiedene Messfrequenzen des Messsignals (Wechselspannungsmesssignals oder des Wechselstrommesssignals) ermittelt. Hierzu wird die Messfrequenz des Messsignals zwischen mehreren Messfrequenzen geändert und für jede der Messfrequenzen wird jeweils ein Impedanzistwert ermittelt. Der Impedanzistwert ist somit frequenzabhängig. Dadurch lässt sich beispielsweise ein frequenzabhängiger Impedanzverlauf zumindest näherungsweise ermitteln.

Die Messfrequenz für das Messsignal kann vorzugsweise aus einem Frequenzbereich von 10 Hz bis 1,0 MHz ausgewählt werden. Beispielsweise kann jeweils ein Impedanzistwert pro Messfrequenz für mindestens 10 oder mindestens 20 oder mindestens 50 unterschiedliche Messfrequenzen ermittelt werden. Vorzugsweise kann zumindest eine Messfrequenz aus dem Bereich von 10 Hz bis 100 Hz ausgewählt werden. Weiter vorzugsweise kann zumindest eine Messfrequenz aus dem Bereich von 100 kHz bis 1,0 MHz ausgewählt werden. Zwei der ausgewählten Messfrequenzen können gleich groß sein wie die Grenzen des gesamten möglichen Frequenzbereichs, so dass beispielsgemäß die kleinste Messfrequenz gleich 10 Hz und die größte Messfrequenz gleich 1,0 MHz ist. In dem angegebenen Messfrequenzbereich beginnend bei 10 Hz können beispielsweise 10 unterschiedlich große Messfrequenzen pro Größenordnung ausgewählt werden. Die Frequenzabstände zwischen zwei Messfrequenzen können gleich groß sein oder exponentiell mit der Frequenz ansteigen.

Die Amplitude des Wechselspannungsmesssignals ist vorzugsweise kleiner als 0,1 V. Bei einem Ausführungsbeispiel kann die Amplitude des Wechselspannungsmesssignals etwa 10 mV betragen.

Die Impedanzistwerte können anhand eines vorgegebenen frequenzabhängigen Prüfkriteriums geprüft werden. Das Prüfkriterium erlaubt somit eine frequenzabhängige Bewertung der ermittelnden Impedanzistwerte. Für jede Messfrequenz kann eine Bewertung des ermittelten Impedanzistwerts durchgeführt werden. Insbesondere lassen sich dadurch kapazitive und ohmsche Einflüsse auf die Impedanzistwerte besser erkennen und unterscheiden. Als Prüfkriterium kann zum Beispiel ein frequenzabhängiger Impedanzsollbereich definiert werden, innerhalb dem die gemessenen Impedanzistwerte liegen müssen. Der Impedanzsollbereich kann dabei einen linear oder nicht linear von der Messfrequenz abhängigen Verlauf aufweisen und ist insbesondere nicht konstant.

Es hat sich herausgestellt, dass durch das Messen und Bewerten der Impedanz der Neutralelektrode bei mehreren Messfrequenzen eine verbesserte Bewertung der elektrischen Verbindung zwischen der Neutralelektrode und dem Patienten erreicht wird. Beispielsweise lassen sich unzureichende elektrische Verbindungen zwischen der Neutralelektrode und dem Patienten unabhängig davon erkennen, welche räumlichen Flächenanteile der Neutralelektrode nicht elektrisch leitend mit dem Patienten verbunden sind. Insbesondere lässt sich ein unzureichender elektrischer Kontakt zwischen der Neutralelektrode und dem Patienten unabhängig davon erkennen, an welcher Stelle oder aus welcher Raumrichtung sich die Neutralelektrode während des Betriebs des Elektrochirurgiesystems vom Patienten ablöst.

Die Messung von Impedanzen bei unterschiedlichen Messfrequenzen ist zwar an sich bekannt, beispielsweise aus der Impedanzspektroskopie. Allerdings soll dadurch die Unterscheidung von Gewebetypen ermöglicht werden, die mittels des elektrochirurgischen Instruments behandelt werden. Ein derartiges Verfahren ist beispielsweise in EP 1 289 415 A1 beschrieben. Mittels dem zur Behandlung verwendeten Instrument wird ein Prüfsignal bei unterschiedlichen Frequenzen an das behandelte Gewebe angelegt und anhand der gemessenen Impedanz wird das behandelte Gewebe charakterisiert, um unterschiedliche Gewebetypen zu erkennen.

EP 0 813 387 A1 offenbart ein Gerät für die Charakterisierung und die Behandlung von Tumoren. Auch hier wird die Impedanz des mit einem Instrument behandelten Gewebes zur Unterscheidung unterschiedlicher Gewebetypen verwendet.

Bei dem aus WO 03/060462 A2 bekannten Verfahren wird ein elektrischer Puls in ein Gewebe eingeleitet und dessen Reflexion erfasst. Anhand der Reflexion soll in Echtzeit gesundes von krankem Gewebe unterschieden werden.

Ein Elektrochirurgiesystem mit einer Messeinheit ist außerdem in EP 3 496 638 A1 beschrieben. Dort wird eine Impedanzmessung bei verschiedenen Frequenzen des behandelnden Gewebes mittels einer Messeinrichtung durchgeführt. Mittels der Messeinrichtung kann ein Messsignal an das Gewebe angelegt werden. Eine Umschalteinrichtung dient dazu, zwischen einer Spannung zur Behandlung des Gewebes und dem Messsignal umzuschalten. Mittels des Messsignals wird die Impedanz des Gewebes bei unterschiedlichen Frequenzen ermittelt.

Ein weiteres Verfahren zur Ermittlung eines lokalen Gewebetyps eines Körpergewebes und ein entsprechendes Elektrochirurgiesystem sind in DE 10 2019 209 333 A1 beschrieben. Zur Gewebebestimmung wird ein Messsignal (Wechselspannung oder Wechselstrom) in das Gewebe eingekoppelt, wobei die Frequenz variieren kann. Basierend darauf kann beispielsweise ein Impedanzspektrum erfasst und daraus der Gewebetyp abgeleitet werden.

Im Stromkreis zwischen dem Versorgungsgerät, dem Instrument, dem Patienten und der Neutralelektrode variiert die Impedanz abhängig von einer Mehrzahl von Parametern, so dass ein Rückschluss auf den elektrischen Kontakt zwischen der Neutralelektrode und dem Patienten nicht möglich ist. Im Unterschied dazu gestattet die vorliegende Erfindung eine gegenüber dem Stand der Technik verbesserte Bewertung der elektrischen Verbindung zwischen der Neutralelektrode und dem Patienten.

Bei einer bevorzugten Ausführungsform weist die Neutralelektrode mehrere, beispielsweise zwei oder drei, elektrisch nicht unmittelbar miteinander verbundene Elektrodenbereiche auf. Die Elektrodenbereiche sind somit nicht kurzgeschlossen. Sie können daher unterschiedliche elektrische Potentiale aufweisen. Innerhalb der Neutralelektrode sind die Elektrodenbereiche in Bezug auf die auftretenden Ströme und Spannungen elektrisch voneinander isoliert und eine elektrische Verbindung besteht vorzugsweise ausschließlich mittelbar über den Patienten, wenn die Neutralelektrode am Patienten angebracht ist. Beispielsweise kann die Neutralelektrode einen elektrisch leitfähigen ersten Elektrodenbereich und einen elektrisch leitfähigen zweiten Elektrodenbereich aufweisen.

Vorzugsweise sind wenigstens zwei der vorhandenen elektrisch leitfähigen Elektrodenbereiche über jeweils einen Leiter mit dem Neutralanschluss des Versorgungsgeräts verbunden. Beispielsweise kann der erste Elektrodenbereich über einen ersten Leiter und der zweite Elektrodenbereich über einen zweiten Leiter mit dem Neutralanschluss elektrisch verbunden sein. Die beiden Leiter sind für die auftretenden Ströme und Spannungen elektrisch gegeneinander isoliert.

Es ist vorteilhaft, wenn der erste Elektrodenbereich und der zweite Elektrodenbereich gleich große Flächeninhalte und/oder dieselbe Geometrie aufweisen. Bei einer bevorzugten Ausführungsform sind die beiden Elektrodenbereiche mit Abstand zueinander angeordnet. Sie können durch einen elektrisch nicht leitfähigen Steg der Neutralelektrode elektrisch voneinander isoliert sein.

Der erste Elektrodenbereich und der zweite Elektrodenbereich können bei einer Ausführungsform auf entgegengesetzten Seiten einer Bezugsebene und relativ zur Bezugsebene symmetrisch angeordnet sein. Die Bezugsebene kann sich entlang des Stegs erstrecken, der die beiden Elektrodenbereiche voneinander trennt.

Die Neutralelektrode weist vorzugsweise einen elektrisch leitfähigen dritten Elektrodenbereich auf. Der dritte Elektrodenbereich ist für die auftretenden Ströme und Spannungen innerhalb der Neutralelektrode elektrisch isoliert gegenüber dem ersten Elektrodenbereich und dem zweiten Elektrodenbereich. Eine elektrische Verbindung zwischen zwei oder mehr Elektrodenbereichen ist ausschließlich mittelbar herstellbar, beispielsweise durch eine elektrisch leitende Verbindung von zwei oder mehr Elektrodenbereichen mit einer elektrisch leitfähigen Struktur, wie zum Beispiel dem Patienten, wenn die Neutralelektrode am Patienten angebracht ist.

Der dritte Elektrodenbereich kann den ersten Elektrodenbereich und den zweiten Elektrodenbereich umschließen. Der dritte Elektrodenbereich ist in seiner Erstreckungsrichtung vorzugsweise in mehreren Abschnitten gekrümmt und/oder abgewinkelt und ist weiter vorzugsweise unterbrechungslos ausgestaltet. Er kann zum Beispiel einen U-förmigen oder C-förmigen Verlauf haben. Der dritte Elektrodenbereich ist bevorzugt lediglich an einer einzigen Stelle offen, an der sich ein Anschlussbereich zur elektrischen Kontaktierung des ersten Elektrodenbereichs und des zweiten Elektrodenbereichs mit dem ersten Leiter bzw. dem zweiten Leiter befindet.

Der Flächeninhalt des dritten Elektrodenbereichs kann kleiner sein als der Flächeninhalt des ersten Elektrodenbereichs und/oder des zweiten Elektrodenbereichs. Die Länge des dritten Elektrodenbereichs entlang seiner Erstreckungsrichtung - vorzugsweise um den ersten Elektrodenbereich und den zweiten Elektrodenbereich herum - hat bei einer bevorzugten Ausführungsform einen Betrag, der zumindest um den Faktor 10 oder 20 größer ist als der Betrag seiner maximalen Breite rechtwinklig zu dieser Erstreckungsrichtung (Länge).

Wie es bereits vorstehend erläutert wurde, kann das Prüfkriterium auf einem oder mehreren vorgegebenen frequenzabhängigen Impedanzvergleichswerten basieren. Beispielsweise kann ein von der Frequenz abhängiger oberer Impedanzgrenzwert und/oder ein von der Frequenz abhängiger unterer Impedanzgrenzwert vorgegeben sein, mit dem oder mit denen die ermittelten Impedanzistwerte verglichen wird bzw. werden. Anhand dieser Prüfung kann erkannt werden, ob die Neutralelektrode den Vorgaben entsprechend mit einem ausreichend elektrisch leitenden Kontakt am Patienten anliegt.

Das Prüfkriterium basiert darauf, dass eine die Neutralelektrode charakterisierende Modellimpedanz vorgegeben wird. Die Modellimpedanz beschreibt die Abhängigkeit der Impedanzistwerte von mehreren Einflussgrößen bzw. Parametern. Zu diesen Parametern gehört auch die räumliche Abhängigkeit der Flächenanteile der Kontaktfläche der Neutralelektrode, die niederohmig mit dem Patienten verbunden sind bzw. der Flächenanteile der Neutralelektrode, die keine ausreichend niederohmige elektrische Verbindung zum Patienten haben. Mithin kann z.B. eine Raumrichtung berücksichtigt werden, aus der sich die Neutralelektrode ausgehend von ihrem Rand vom Patienten ablöst.

Vorzugsweise weist die Modellimpedanz eine Warburg-Impedanz und/oder ein Konstantphasenelement (englisch: Constant Phase Element, CPE) auf.

Die Warburg-Impedanz kann innerhalb der Modellimpedanz mehrere Parameter zur Charakterisierung des Verhaltens der Neutralelektrode bereitstellen, beispielsweise einen Gleichspannungswiderstand und eine Zeitkonstante. Der Gleichspannungswiderstand und die Zeitkonstante können durch Simulation und/oder empirisch ermittelt werden und hängen von der jeweiligen Ausgestaltung der Neutralelektrode ab.

Das Konstantphasenelement ist ein Element, das als vorgebbaren Parameter eine konstante Phasenverschiebung definiert, insbesondere im Bereich von 0° bis -90° und kann einen kapazitiven Einfluss charakterisieren. Das Konstantphasenelement kann abhängig von der Vorgabe der durch den wählbaren Parameter definierten Phasenverschiebung einen ausschließlich ohmschen Widerstand (Phasenverschiebung 0°), einen idealen Kondensator (Phasenverschiebung -90°) oder eine Kombination davon beschreiben.

Die Modellimpedanz hat bei einem Ausführungsbeispiel eine Parallelschaltung, wobei die Warburg-Impedanz und das Konstantphasenelement parallel zueinander geschaltet sind.

Die Modellimpedanz kann außerdem wenigstens einen Widerstand aufweisen. Bei einer bevorzugten Ausführungsform sind zwei Widerstände vorhanden. Der eine Widerstand ist innerhalb der Parallelschaltung in Reihe zu der Warburg-Impedanz geschaltet, während der andere Widerstand in Reihe zur Parallelschaltung geschaltet sein kann. Somit kann die Modellimpedanz einem Randles-Schaltkreis ("Randles-Circuit") entsprechen.

Mittels der Modellimpedanz werden mehrere (z.B. fünf) Parameter oder Variablen bereitgestellt, die durch Simulation und/oder Messung definiert werden können, um die Neutralelektrode zu beschreiben im Hinblick auf die Frequenzabhängigkeit der Impedanz sowie die räumliche Anordnung der Flächenanteile der Neutralelektrode, die elektrisch leitend am Patienten anliegen.

Zusätzlich oder alternativ zu einem Prüfkriterium, das auf wenigstens einem frequenzabhängigen Impedanzvergleichswert basiert, kann auch eine Klassifizierung unter Verwendung von wenigstens einem Klassifikator durchgeführt werden. Der Klassifikator kann dabei unmittelbar auf die ermittelten Impedanzistwerte angewandt werden und/oder auf einen oder mehrere Ersatzparameter angewandt werden, die basierend auf den ermittelten Impedanzistwerten und/oder der Modellimpedanz ermittelt wurden. Solche Ersatzparameter können beispielsweise dadurch erhalten werden, dass sämtliche verfügbare Variablen bzw. Parameter durch ein Verfahren der Dimensionsreduktion zu einem oder mehreren Ersatzparametern umgewandelt werden. Verfahren der Dimensionsreduktion sind bekannt, beispielsweise in Form der Hauptkomponentenanalyse (HKA, englisch: Principle Component Analysis, PCA) oder durch mathematische bzw. statistische Verfahren, wie z.B. t-Distributed Stochastic Neighbor Embedding, t-SNE).

Als Klassifikatoren können sämtliche bekannte Klassifikatoren verwendet werden, beispielsweise einer oder mehrere der folgenden Klassifikatoren: Bayes-Klassifikator, ein Stützvektorverfahren (Support Vector Machine, SVM), ein künstliches neuronales Netz (beispielsweise ein faltendes neuronales Netz, englisch: Convolutional Neural Network, CNN), Entscheidungsbäume (englisch: Decision Tree) und/oder ein k-nächste-Nachbarn-Algorithmus (englisch: k-Nearest-Neighbor-Algorithm, KNN).

Beispielsweise kann ein künstliches neuronales Netz zwei Eingangsneuronen für jeden Impedanzistwert, d.h. pro vorgegebener Messfrequenz aufweisen, so dass sich die beiden Dimensionen (Betrag/ Phase oder Realteil/ Imaginärteil) des jeweiligen Impedanzistwerts in die Eingangsschicht des künstlichen neuronalen Netzwerkes zuführen lassen. Das künstliche neuronale Netzwerk kann basierend auf Trainingsdaten trainiert werden, um die unterschiedlichen Fallgestaltungen zu erlernen, in denen kein ausreichend elektrisch leitender Kontakt zwischen der Neutralelektrode und dem Patienten vorhanden ist. Die Trainingsdaten können aus tatsächlichen Betriebsdaten und/oder aus Simulationen und/oder Versuchen im Labor gewonnen werden.

Bei einer bevorzugten Ausführungsform hat das Elektrochirurgiesystem einen Versorgungsanschluss, an den ein elektrochirurgisches Instrument zur Behandlung von Gewebe eines Patienten anschließbar ist. An den Versorgungsanschluss kann das Versorgungsgerät eine Arbeitsspannung und/oder einen Arbeitsstrom für das Instrument anlegen. Die Arbeitsspannung bzw. der Arbeitsstrom können abhängig von der chirurgischen Anwendung durch das Versorgungsgerät vorgegeben werden, beispielsweise abhängig davon, ob mittels des Instruments Gewebe koaguliert, geschnitten, abladiert oder fusioniert werden soll.

Das Instrument hat eine Arbeitselektrode. Basierend auf der Arbeitsspannung kann an die Arbeitselektrode eine Elektrodenspannung angelegt werden und/oder basierend auf dem Arbeitsstrom kann ein Elektrodenstrom durch Arbeitselektrode und weiter in das zu behandelnde Gewebe fließen. Als Elektrodenspannung kann die Arbeitsspannung und/oder als Elektrodenstrom kann der Arbeitsstrom verwendet werden.

Bei einem Ausführungsbeispiel ist das Instrument dazu eingerichtet, die entsprechende Arbeitsspannung bzw. den entsprechenden Arbeitsstrom am Versorgungsanschluss anzufordern. Hierfür kann ein Bedienelement des Instruments über eine Signalleitung mit dem Versorgungsanschluss verbunden sein. Zusätzlich oder alternativ kann der Strompfad zwischen dem Versorgungsanschluss und der Arbeitselektrode durch ein Bedienelement freigegeben oder unterbrochen oder umgeschaltet werden. Dabei ist es optional auch möglich, die bereitgestellte Arbeitsspannung bzw. den bereitgestellten Arbeitsstrom innerhalb des elektrischen Pfades vom Versorgungsanschluss bis zur Arbeitselektrode zu modifizieren, beispielsweise über eine Wandlerschaltung, so dass die Elektrodenspannung und/oder der Elektrodenstrom verschieden sind von der Arbeitsspannung bzw. dem Arbeitsstrom. Eine solche Modifikation kann beispielsweise auch zum Erzeugen einer weiteren Betriebsart außerhalb des Versorgungsgeräts verwendet werden.

Das Elektrochirurgiesystem kann bei einer Ausführungsform dazu eingerichtet sein, das Wechselspannungsmesssignal nur dann an den Neutralanschluss und mithin die Neutralelektrode anzulegen, wenn an der Arbeitselektrode keine Elektrodenspannung anliegt und/oder wenn kein Elektrodenstrom durch die Arbeitselektrode in das Gewebe fließt. Somit kann das Wechselspannungsmesssignal zur Ermittlung von einem oder mehreren Impedanzistwerten in einem oder mehreren Zeiträumen zwischen Behandlungsperioden an den Neutralanschluss bzw. die Neutralelektrode angelegt werden. Dadurch wird der Messstrom nicht durch die Arbeitsspannung bzw. den Arbeitsstrom beeinflusst und die Ermittlung der Impedanzistwerte ist unabhängig von der Arbeitsspannung und dem Arbeitsstrom.

Zusätzlich oder alternativ hierzu ist es auch möglich, zumindest phasenweise gleichzeitig Gewebe mittels der Arbeitselektrode zu behandeln (an der Arbeitselektrode liegt eine Elektrodenspannung an und/oder es fließt ein Arbeitsstrom durch die Arbeitselektrode) und das Wechselspannungsmesssignal an den Neutralanschluss bzw. die Neutralelektrode anzulegen. In den Zeiträumen, in denen sich eine Behandlungsperiode und das Anlegen des Wechselspannungsmesssignals überlappen, kann die Messfrequenz des Wechselspannungsmesssignals verschieden gewählt werden von der Frequenz der Arbeitsspannung und/oder Arbeitsstromes und/oder der Elektrodenspannung und/oder des Elektrodenstromes. Dadurch ist sichergestellt, dass sich der durch das Wechselspannungsmesssignal ergebende Messstrom durch die Neutralelektrode vom Elektrodenstrom und einem dadurch verursachten Stromfluss durch das Gewebe unterscheiden lässt - beispielsweise durch Auswertung der Frequenz. Auch in diesem Fall ist eine ausreichend genaue Bestimmung der Impedanzistwerte möglich.

Bei dem erfindungsgemäßen Verfahren zum Prüfen der elektrischen Verbindung zwischen der Neutralelektrode und dem Patienten wird die Neutralelektrode an den Patienten angelegt, um eine elektrisch leitende Verbindung mit dem Patienten herzustellen. Anschließend wird ein Messsignal in Form eines Wechselspannungsmesssignals oder eines Wechselstrommesssignals an die Neutralelektrode angelegt bzw. eingeprägt - insbesondere über den Neutralanschluss des Versorgungsgeräts eines Elektrochirurgiesystems gemäß einem der vorhergehenden Ausführungsbeispiele. Dadurch wird ein Messstrom oder eine Messspannung erzeugt, der bzw. die gemessen werden kann. Somit kann wiederum basierend auf dem angelegten Wechselspannungsmesssignal und dem Messstrom, bzw. dem angelegten Wechselstrommesssignal und der Messspannung ein Impedanzistwert ermittelt werden (Ohmsches Gesetz). Das Messsignal (Wechselspannungsmesssignal oder Wechselstrommesssignal) wird insbesondere nacheinander bei mehreren unterschiedlichen Messfrequenzen erzeugt und jeweils ein Impedanzistwert für jede ausgewählte und eingestellte Messfrequenz ermittelt. Die auf diese Weise erhaltenen Impedanzistwerte, die einen durch die Messfrequenzen definierten Frequenzbereich abdecken, können anschließend anhand eines vorgegebenen Prüfkriteriums geprüft werden. Diese Prüfung liefert ein Prüfergebnis, das angibt, ob eine ausreichende elektrische Verbindung zwischen der Neutralelektrode und dem Patienten besteht.

Vorteilhafte Ausführungen der Erfindung ergeben sich aus den abhängigen Ansprüchen, der Zeichnung und der Beschreibung. Nachfolgend werden bevorzugte Ausführungsbeispiele anhand der beigefügten Zeichnungen im Einzelnen erläutert. In der Zeichnung zeigen:
Figur 1 eine schematische blockschaltbildähnliche Darstellung eines Ausführungsbeispiels eines Elektrochirurgiesystems aufweisend ein Versorgungsgerät, eine Neutralelektrode und ein Instrument,
Figur 2 eine schematische Prinzipdarstellung eines Ausführungsbeispiels einer Neutralelektrode,
Figur 3 eine schematische Prinzipdarstellung von Übergangswiderständen zwischen Elektrodenflächen der Neutralelektrode in Figur 2,
Figur 4 beispielhafte prinzipielle Änderungen eines Gesamtwiderstands der Neutralelektrode gemäß Figuren 2 und 3 abhängig von einem Flächenanteil der gesamten, verfügbaren Kontaktfläche der Neutralelektrode, der in elektrisch leitendem Kontakt mit dem Patienten steht,
Figur 5 ein Blockschaltbild einer Modellimpedanz, die die Neutralelektrode gemäß der Figuren 1 und 2 charakterisiert,
Figur 6 ein Blockschaltbild des Ausführungsbeispiels des Elektrochirurgiesystems nach Figur 1,
Figur 7 einen beispielhaften Impedanzverlauf basierend auf mehreren ermittelten Impedanzistwerten der Neutralelektrode gemäß der Figuren 1 und 2,
Figur 8 schematisch und lediglich beispielhaft dargestellte Felder, die den Flächenanteil der verfügbaren Kontaktfläche definieren, der elektrisch leitend mit dem Patienten verbunden ist abhängig von zwei Hauptkomponenten,
Figur 9 beispielhafte zeitliche Verläufe einer Arbeitsspannung und mehrerer möglicher Wechselspannungsmesssignale,
Figur 10 stark schematisiert dargestellte Spektren eines Arbeitsstromes und eines Messstromes und
Figur 11 ein Flussdiagramm eines Ausführungsbeispiels eines erfindungsgemäßen Verfahrens.

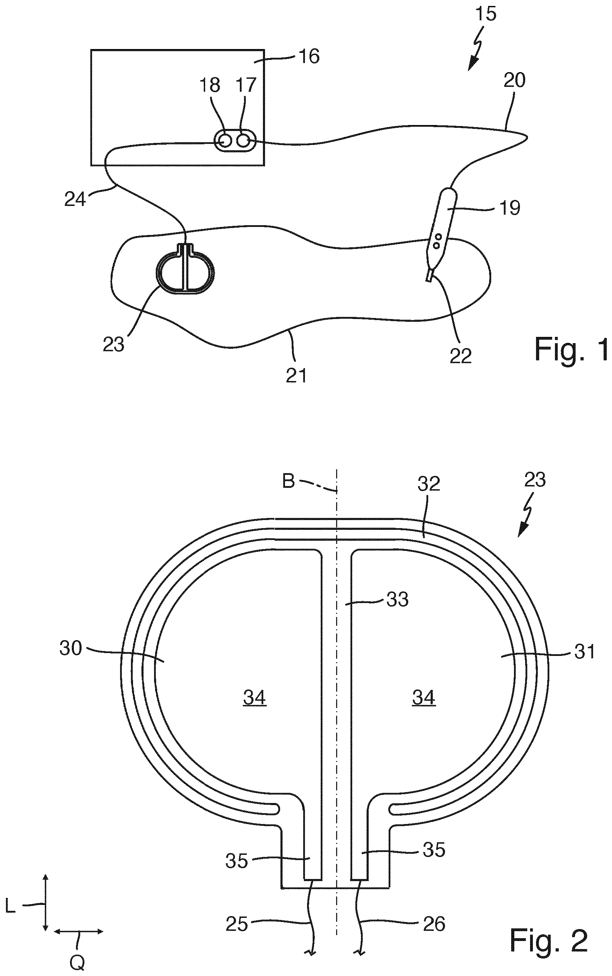

In Figur 1 ist eine Prinzipdarstellung eines Ausführungsbeispiels eines Elektrochirurgiesystems 15 veranschaulicht. Das Elektrochirurgiesystem 15 hat ein Versorgungsgerät 16 mit einem Versorgungsanschluss 17 sowie einem Neutralanschluss 18. Der Versorgungsanschluss 17 dient dazu, ein Instrument 19 elektrisch mit dem Versorgungsgerät 16 zu verbinden. Die elektrische Verbindung kann einpolig oder mehrpolig sein. Die Verbindung kann somit über ein einadriges oder mehradriges Kabel 20 hergestellt werden.

Das Instrument 19 ist zur elektrochirurgischen Behandlung von biologischem Gewebe 21 eines Patienten eingerichtet. Das Gewebe 21 ist biologisches Gewebe eines menschlichen oder tierischen Körpers. Zur Behandlung des Gewebes 21 hat das Instrument 19 wenigstens eine oder genau eine Arbeitselektrode 22. Zur Behandlung des Gewebes 21 kann eine elektrisch leitende Verbindung zwischen der Arbeitselektrode 22 und dem Gewebe 21 hergestellt werden.

Bei dem Elektrochirurgiesystem 15 gemäß Figur 1 ist das Instrument 19 als monopolares Instrument ausgebildet. Ein Stromkreis zwischen dem Versorgungsgerät 16, der Arbeitselektrode 22 des Instruments 19 und wieder zurück zum Versorgungsgerät 16 wird nicht ausschließlich über das Instrument 19, sondern zusätzlich über eine separate Elektrode hergestellt, die am Patienten elektrisch leitend angebracht wird. Diese zusätzliche Elektrode kann als Neutralelektrode 23 bezeichnet werden. Die Neutralelektrode 23 ist beispielsgemäß über ein mehradriges Kabel 24 mit dem Neutralanschluss 18 des Versorgungsgeräts 16 elektrisch verbunden. Das Kabel 24 hat zum Beispiel zumindest zwei Adern oder Leiter, und beim dargestellten Ausführungsbeispiel einen ersten Leiter 25 und einen zweiten Leiter 26 (Figur 2).

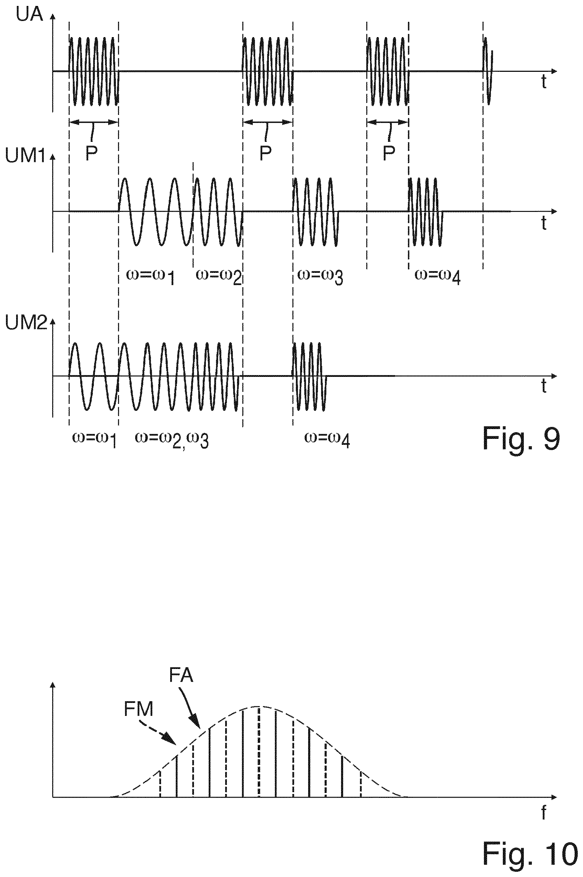

Das Versorgungsgerät 16 kann an dem Versorgungsanschluss 17 eine Arbeitsspannung UA (Figur 9) für die Arbeitselektrode 22 anlegen. Bei elektrisch leitendem Kontakt zwischen der Arbeitselektrode 22 und dem Gewebe 21 fließt ein Arbeitsstrom IA durch die Arbeitselektrode 22, weiter durch das Gewebe 21 zur Neutralelektrode 23 und von dort zum Neutralanschluss 18 des Versorgungsgeräts 16. Der Stromkreis ist geschlossen.

Die Arbeitsspannung UA ist eine Hochfrequenzspannung, die während dem Instrument 19 zur Behandlung des Gewebes 21 bereitgestellt wird und bei einem Ausführungsbeispiel als Elektrodenspannung an die Arbeitselektrode 22 angelegt werden kann. Optional kann die am Versorgungsanschluss 17 bereitgestellte Arbeitsspannung UA durch einen Wechselrichterschaltung oder eine andere Modifikationsschaltung in eine geeignete Elektrodenspannung umgewandelt werden. Beim Ausführungsbeispiel wird mittels des Instruments 19 über eine Signalleitung des Kabels 20 die Arbeitsspannung UA am Versorgungsanschluss 17 angefordert. Die Arbeitsspannung UA liegt dann während Behandlungsperioden P am Versorgungsanschluss 17 an der Arbeitselektrode 22 an, wie es schematisch in Figur 9 dargestellt ist.

Ein Ausführungsbeispiel einer Neutralelektrode 23 ist in Figur 2 dargestellt. Die Neutralelektrode 23 hat beispielsgemäß mehrere elektrisch leitfähige Elektrodenbereiche und bei der bevorzugten Ausführungsform einen elektrisch leitfähigen ersten Elektrodenbereich 30, einen elektrisch leitfähigen zweiten Elektrodenbereich 31 und einen elektrisch leitfähigen dritten Elektrodenbereich 32. Die Elektrodenbereiche 30, 31, 32 sind mit Abstand zueinander angeordnet und für die auftretenden Spannungen und Ströme elektrisch voneinander isoliert, so dass jeder Elektrodenbereich 30, 31, 32 ein anderes elektrisches Potential aufweisen kann. In am Patienten angebrachtem Zustand sind die Elektrodenbereiche 30, 31, 32 ausschließlich mittelbar über den Patienten elektrisch miteinander verbunden. Beispielsweise können die Elektrodenbereiche 30, 31, 32 mit Abstand zueinander auf einem elektrisch nicht leitenden Träger der Neutralelektrode 23 angeordnet sein.

Die Elektrodenbereiche 30, 31, 32 sind nur auf der dem Patienten zugewandten Seite als elektrisch leitfähige Kontaktfläche 34 ausgebildet und auf der jeweils anderen Seite durch ein Trägermaterial oder anderweitiges Isolationsmaterial der Neutralelektrode 23 elektrisch isoliert.

Der erste Elektrodenbereich 30 und der zweite Elektrodenbereich 31 weisen denselben Flächeninhalt und dieselbe Geometrie auf. Bezüglich einer Bezugsebene B durch die Neutralelektrode 23 sind der erste Elektrodenbereich 30 und der zweite Elektrodenbereich 31 symmetrisch angeordnet. Die Bezugsebene B erstreckt sich rechtwinklig zu einer Querrichtung Q der Neutralelektrode 23. In einer Längsrichtung L parallel zur Bezugsebene B verläuft ein elektrisch nicht leitfähiger Steg 33 zwischen dem ersten Elektrodenbereich 30 und dem zweiten Elektrodenbereich 31.

Der erste Elektrodenbereich 30 und der zweite Elektrodenbereich 31 können im Wesentlichen eine jeweils halbkreisförmige Kontaktfläche 34 aufweisen, die zur Anlage mit dem Patienten eingerichtet ist. Der erste Elektrodenbereich 30 und der zweite Elektrodenbereich 31 weisen jeweils einen Anschlussbereich 35 auf, der durch eine leiterbahnähnliche Verlängerung der betreffenden Kontaktfläche 34 ausgebildet ist. Ausgehend von der Kontaktfläche 34 kann sich jeder Anschlussbereich 35 im Wesentlichen in Längsrichtung L erstrecken. An den Anschlussbereichen 35 sind der erste Elektrodenbereich 30 mit dem ersten Leiter 25 und der zweite Elektrodenbereich 31 mit dem zweiten Leiter 26 elektrisch verbunden.

Der dritte Elektrodenbereich 32 ist nicht elektrisch mit dem Versorgungsgerät 16 verbunden. Sein elektrisches Potential ist daher nicht mittels dem Versorgungsgerät 16 definiert.

Der dritte Elektrodenbereich 32 erstreckt sich außerhalb der Anschlussbereiche 35 vollständig um den ersten Elektrodenbereich 30 und den zweiten Elektrodenbereich 31 herum. Der Flächeninhalt des dritten Elektrodenbereichs 32 ist vorzugsweise kleiner als der Flächeninhalt des ersten Elektrodenbereichs 30 und des zweiten Elektrodenbereichs 31. Entlang seiner Erstreckungsrichtung bzw. in Umfangsrichtung um den ersten Elektrodenbereich 30 und den zweiten Elektrodenbereich 31 hat der dritte Elektrodenbereich 32 eine Länge, deren Betrag deutlich größer ist als der Betrag seiner Breite rechtwinklig zu seiner Erstreckungsrichtung betrachtet. Der dritte Elektrodenbereich 32 kann somit linienförmig oder leiterbahnförmig sein. Abgesehen von den Anschlussbereichen 35 ist der dritte Elektrodenbereich 32 zusammenhängend unterbrechungslos ausgebildet.

Beim Ausführungsbeispiel ist der dritte Elektrodenbereich 32 der einzige Elektrodenbereich, der sich durch die Bezugsebene B hindurch erstreckt. Er kann zwei bogenförmige und insbesondere kreisbogenförmige Endabschnitte aufweisen, die über einen vorzugsweise geradlinigen Verbindungsabschnitt miteinander verbunden sind.

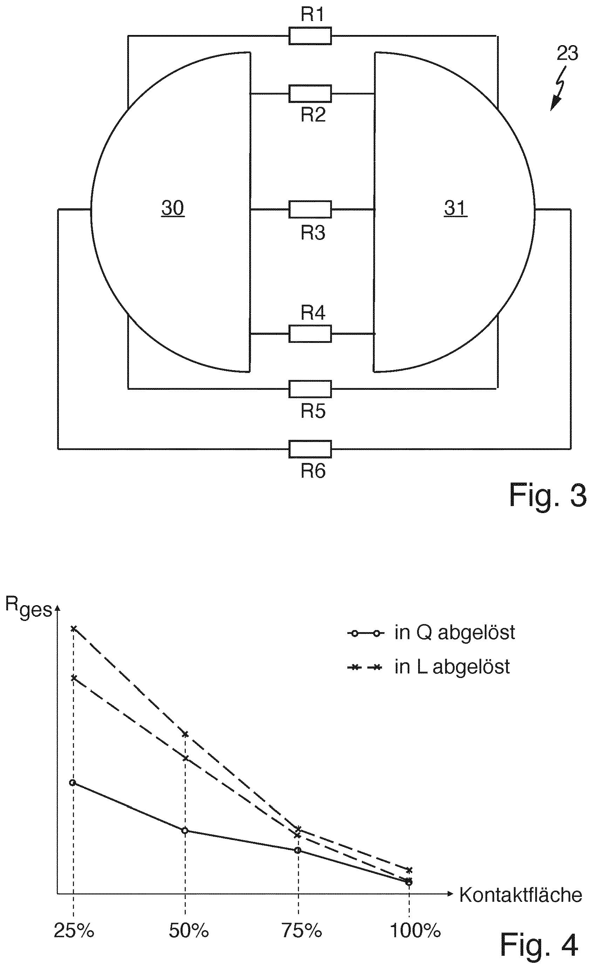

Die in Figur 2 dargestellte Neutralelektrode weist in an einem Patienten angebrachter Gebrauchslage unterschiedliche elektrische Übergangswiderstände Rₖ (k=1,2,...,n) zwischen dem ersten Elektrodenbereich 30 und dem zweiten Elektrodenbereich 31 auf, die abhängig von der räumlichen Position (also abhängig von der Position in Längsrichtung L und Querrichtung Q) variieren. Die Übergangswiderstände Rₖ (k=1,2,...,n) variieren abhängig davon, zwischen welchen Stellen an den Kontaktflächen 34 bzw. Elektrodenbereichen 30, 31 der Übergangswiderstand Rₖ gemessen wird. In der Prinzipskizze nach Figur 3 sind beispielhaft sechs Übergangswiderstände R₁ bis R₆ dargestellt. Die Übergangswiderstände (im Beispiel: R₁, R₅ und R₆), die zwischen den nicht an den Steg 33 angrenzenden, abgewandten Kanten der Elektrodenbereiche 30, 31 wirksam sind, sind deutlich größer als die Übergangswiderstände (im Beispiel: R₂, R₃ und R₄) zwischen den Kanten der Elektrodenbereiche 30, 31, die unmittelbar an den Steg 33 angrenzen. Dabei kann näherungsweise folgendes angenommen werden: ${R}_{1} \approx {R}_{5} \approx {R}_{6} \approx {R}_{a}$ (1) ${R}_{2} \approx {R}_{3} \approx {R}_{4} \approx {R}_{i}$ (2) ${R}_{a} ≫ {R}_{i}$ (3) Als Folge dieser unterschiedlich großen Übergangswiderstände Rₐ und Rᵢ ändert sich der Gesamtwiderstand R_{ges} zwischen dem ersten Elektrodenbereich 30 und dem zweiten Elektrodenbereich 31 bei einer Reduzierung des Flächeninhalts der Kontaktfläche 34 nicht nur abhängig vom Flächenanteil der Kontaktfläche 34, der tatsächlich elektrisch leitend am Patienten angebracht ist, sondern auch in Abhängigkeit davon, wo dieser Flächenanteil an der Neutralelektrode 23 lokalisiert ist - also beispielsweise in Abhängigkeit davon, ob eine sich die Neutralelektrode vom Patienten in Längsrichtung L oder in Querrichtung Q abgelöst hat.

In Figur 4 sind lediglich beispielhaft und schematisch Messungen für ein Ablösen der Neutralelektrode 23 in Querrichtung Q (durchgezogene Linie in Figur 4) und in Längsrichtung L (gestrichelte Linien in Figur 4) dargestellt. Daraus ist zu erkennen, dass der Gesamtwiderstand R_{ges} zwischen dem ersten Elektrodenbereich 30 und dem zweiten Elektrodenbereich 31 nicht ohne Weiteres einen Rückschluss auf den tatsächlich elektrisch leitend mit dem Patienten verbundenen Flächenanteil der Kontaktfläche 34 zulässt. Als Gesamtwiderstand R_{ges} wurde hier als Realteil der Impedanz der elektrischen Verbindung zwischen dem ersten Leiter 25 und dem zweiten Leiter 26 über das Gewebe 21 ermittelt.

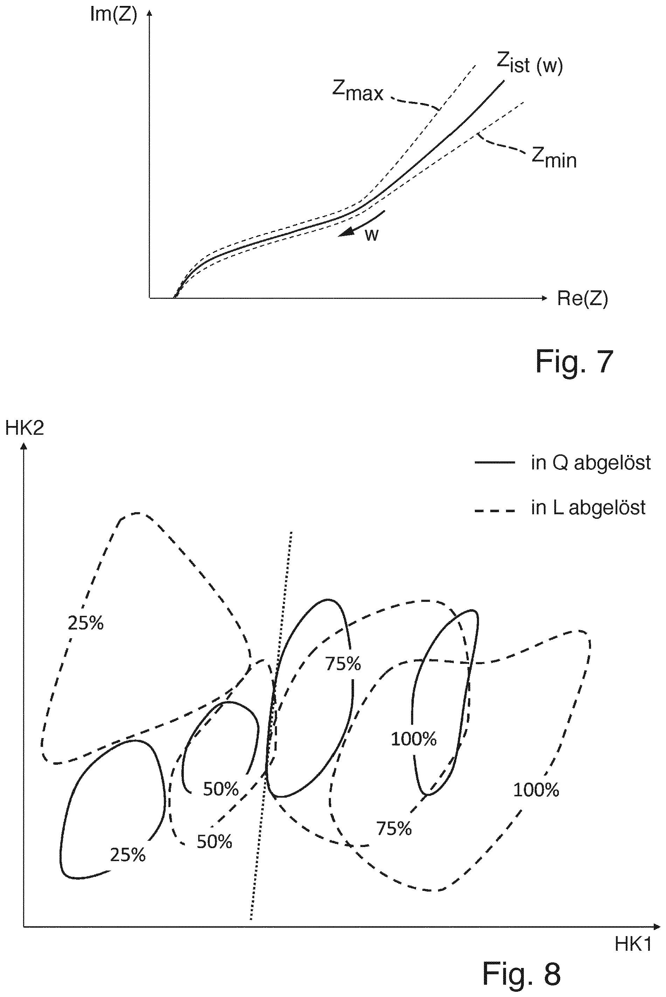

Erfindungsgemäß soll eine verbesserte Überwachung der elektrischen Verbindung zwischen der Neutralelektrode 23 und dem Patienten erreicht werden. Deswegen ist vorgesehen, die Impedanz Z zwischen dem ersten Elektrodenbereich 30 und dem zweiten Elektrodenbereich 31 durch ein Wechselspannungsmesssignal US oder ein Wechselstrommmesssignal IS bei unterschiedlichen Messfrequenzen ω zu ermitteln. Daraus kann beispielsweise ein frequenzabhängiger Impedanzverlauf aus mehreren Impedanzistwerten Zᵢₛₜ ermittelt werden. In Figur 7 sind die Impedanzistwerte Zᵢₛₜ der Realteil Re(Z) und der Imaginärteil Im(Z) der Impedanzistwerte Zᵢₛₜ abhängig von der Messfrequenz ω in Form eines Nyquistdiagramms dargestellt (Messfrequenz ω nimmt beginnend vom Schnittpunkt mit der Abszisse zu).

Wie es ebenfalls in Figur 7 zu erkennen ist, kann der frequenzabhängige Impedanzverlauf oder können einzelne Impedanzistwerte Zᵢₛₜ daraufhin geprüft werden, ob sie innerhalb eines zulässigen Bereichs liegt bzw. liegen. Basierend auf diesem Prüfkriterium kann entschieden werden, ob ein ausreichend guter elektrischer Kontakt zwischen der Neutralelektrode 23 und dem Patienten gegeben ist und somit ein ausreichend großer Flächenanteil der verfügbaren Kontaktfläche 34 der Elektrodenbereiche 30, 31 elektrisch leitend am Patienten anliegt. Beim Ausführungsbeispiel nach Figur 7 besteht das Prüfkriterium somit im Vergleich der gemessenen Impedanzistwerte Zᵢₛₜ mit einem oder mehreren frequenzabhängigen Impedanzvergleichswerten. Als Impedanzvergleichswert kann beispielsweise ein oberer Impedanzgrenzwert Zₘₐₓ und/oder ein unterer Impedanzgrenzwert Zₘᵢₙ vorgegeben sein. Wenn festgestellt wird, dass einer oder mehrere gemessene Impedanzistwerte Zᵢₛₜ nicht innerhalb des vorgegebenen Bereichs liegen, kann auf eine nicht ordnungsgemäße elektrische Verbindung zwischen der Neutralelektrode 23 und dem Patienten geschlossen werden.

Durch das Auswerten der Impedanz Z des Neutralelektrodenstromkreises, also der elektrischen Verbindung zwischen dem ersten Elektrodenbereich 30 und dem zweiten Elektrodenbereich 31 bzw. dem ersten Leiter 25 und dem zweiten Leiter 26, mit einem Wechselspannungsmesssignal US oder einem Wechselstrommesssignal IS bei unterschiedlichen Messfrequenzen ω kann unabhängig von der räumlichen Position an der Neutralelektrode 23 genauer festgestellt werden, ob die Neutralelektrode 23 mit einem ausreichend großen Flächenanteil ihrer wenigstens einen Kontaktfläche 34 eng am Patienten anliegt und mithin die elektrische Verbindung den Vorgaben entspricht. Bei der erfindungsgemäßen Auswertung spielt es keine Rolle, ob sich die Neutralelektrode 23 beispielsweise in Längsrichtung, in Querrichtung oder schräg zu diesen Richtungen vom Patienten ablöst. In allen Fällen lässt sich mit ausreichender Genauigkeit die Qualität der elektrischen Verbindung bewerten.

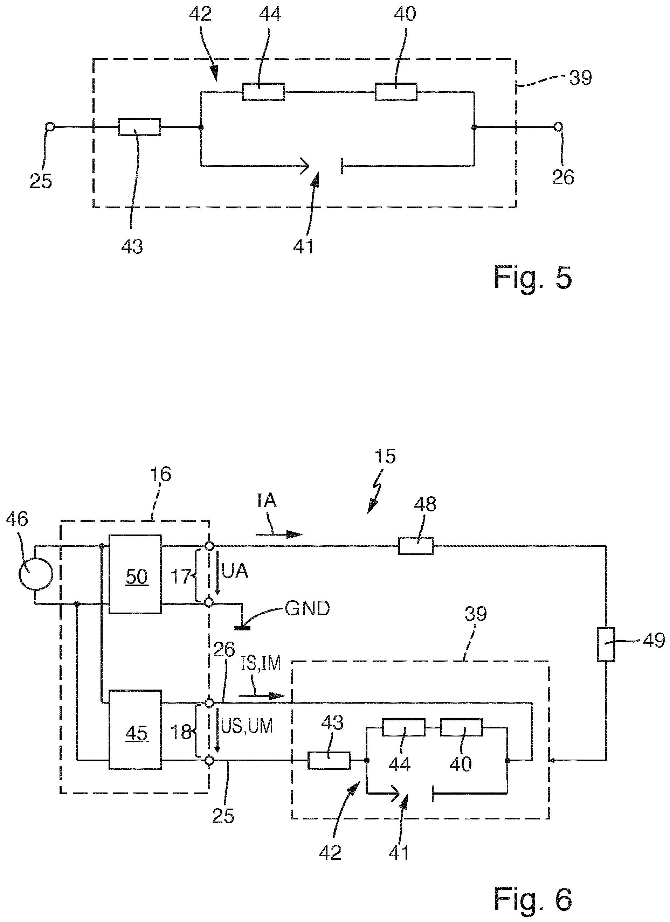

Der Erfindung liegt auch die Erkenntnis zugrunde, dass die Impedanz des Neutralelektrodenstromkreises zwischen dem ersten Elektrodenbereich 30 und dem zweiten Elektrodenbereich 31 durch eine Modellimpedanz 39 darstellen lässt, die in den Figuren 5 und 6 in Form eines Blockschaltbilds veranschaulicht ist. Die Modellimpedanz 39 weist beispielsgemäß eine Warburg-Impedanz 40 auf. Die Warburg-Impedanz hat einen frequenzabhängigen Impedanzwert W (ω) : $W \left(ω\right) = \frac{{A}_{W}}{\sqrt{ω}} + \frac{{A}_{W}}{j \sqrt{ω}}$ (4) wobei A_{w} der Warburgkoeffizient der Warburg-Impedanz 40 ist und ω die Kreisfrequenz ist. Der Warburgkoeffizient A_{W} lässt sich durch Messung und/oder durch Simulation ermitteln und kann insbesondere von der Dimension und/oder Geometrie der Neutralelektrode 23 abhängen.

Zusätzlich zu der Warburg-Impedanz 40 weist die Modellimpedanz 39 beispielsgemäß ein Konstantphasenelement (englisch: Constant Phase Element, CPE) auf. Das Konstantphasenelement 41 ist ein Bauelement, das eine konstante Phasenverschiebung in einem Bereich von 0° bis -90° aufweist. Das Konstantphasenelement 41 kann daher einem idealen Kondensator, einem ohmschen Widerstand oder einer Kombination davon entsprechen.

Wie es in den Figuren 5 und 6 dargestellt ist, weist die Modellimpedanz 39 eine Parallelschaltung 42 und einen ersten Widerstand 43 in Reihe zur Parallelschaltung 42 auf. In der Parallelschaltung 42 ist das Konstantphasenelement 41 parallel zur Warburg-Impedanz 40 geschaltet. In der Parallelschaltung kann in Reihe zur Warburg-Impedanz 40 ein zweiter Widerstand 44 geschaltet sein.

Die Modellimpedanz 39 entspricht beim Ausführungsbeispiel daher einem Randles-Schaltkreis (englisch: Randles Circuit).

Die Modellimpedanz 39 bildet das Verhalten der Neutralelektrode 23 mit ausreichend guter Näherung bzw. Genauigkeit ab.

Zur Messung des Impedanzistwerts Zᵢₛₜ wird an den Neutralanschluss 18 des Versorgungsgeräts 16 das Messsignal (Wechselspannungsmesssignal US angelegt oder ein Wechselstrommesssignal IS) angelegt bzw. eingeprägt und über das Kabel 24 an die Neutralelektrode 23 geleitet. Das Messsignal US, IS ist dadurch zwischen dem ersten Leiter 25 und dem zweiten Leiter 26 und mithin zwischen dem ersten Elektrodenbereich 30 und dem zweiten Elektrodenbereich 31 der Neutralelektrode 23 wirksam. Über die an den Patienten angelegte Neutralelektrode 23 wird der Stromkreis zwischen dem ersten Elektrodenbereich 30 und dem zweiten Elektrodenbereich 31 geschlossen. Durch das Wechselspannungsmesssignal US fließt ein Messstrom IM durch die Neutralelektrode 23 bzw. das Wechselstrommesssignal IS erzeugt über dem Neutralanschluss 18 eine Messspannung UM. Dieser Messstrom IM bzw. diese Messspannung UM kann im Versorgungsgerät 16 erfasst werden, beispielsweise in einer Messeinheit 45 des Versorgungsgeräts 16 (Figur 6). Basierend auf dem Wechselspannungsmesssignal US und dem Messstrom IM oder basierend auf dem Wechselstrommesssignal IS und der Messspannung UM kann die Impedanz bzw. der jeweilige Impedanzistwert Zᵢₛₜ für die jeweils aktuell eingestellte Messfrequenz ω des betreffenden Messsignals US, IS ermittelt werden.

Wie es in Figur 6 ebenfalls veranschaulicht ist, kann das Versorgungsgerät 16 an eine externe Energieversorgung, beispielsweise eine Netzspannungsquelle 46 angeschlossen werden. Die Messeinheit kann dazu eingerichtet sein, aus der Versorgungsspannung der Netzspannungsquelle 46 das Messsignal US, IS zu erzeugen.

Im Ersatzschaltbild gemäß Figur 6 ist die Impedanz, die durch das Instrument 19, die Arbeitselektrode 22 und den Übergang von der Arbeitselektrode 22 zum Gewebe 21 gebildet ist, durch eine erste Impedanz 48 dargestellt. In Reihe dazu stellt eine zweite Impedanz 49 die Impedanz von der Kontaktstelle der Arbeitselektrode 22 mit dem Gewebe 21 bis zur Neutralelektrode 23 und die Übergangsimpedanz zwischen dem Gewebe 21 und der Neutralelektrode 23 dar.

Wie es bereits erläutert wurde, wird am Versorgungsanschluss 17 eine Arbeitsspannung UA und/oder ein Arbeitsstrom IA für das Instrument 19 bereitgestellt. Bei dem in Figur 6 dargestellten Beispiel wird die Arbeitsspannung UA als Elektrodenspannung an die Arbeitselektrode 22 angelegt. Die Arbeitsspannung UA kann durch eine Wechselrichterschaltung 50 des Versorgungsgeräts 16 bereitgestellt werden, beispielsweise aus der Versorgungsspannung der Netzspannungsquelle 46. Zusätzlich oder alternativ ist es auch möglich, eine Wechselrichterschaltung im Instrument 19 oder an einer anderen geeigneten Stelle zwischen dem Versorgungsanschluss 17 und der Arbeitselektrode 22 anzuordnen, um basierend auf der Arbeitsspannung UA eine für die entsprechende Behandlung geeignete Elektrodenspannung zu erzeugen.

Die Arbeitsspannung UA entspricht einer Potentialdifferenz zwischen einem Arbeitspotential und einem Bezugspotential, beispielsweise Masse GND.

In Figur 9 sind schematisch und beispielhaft zeitliche Verläufe für die Arbeitsspannung UA sowie das Messsignal US, IS dargestellt, wobei zur Unterscheidbarkeit in Figur 9 ein erstes Messsignal M1 und ein zweites Messsignal M2 eingeführt wird. Diese Unterscheidung dient lediglich zur Klarheit, um die nachfolgend beschriebenen Möglichkeiten besser voneinander unterscheiden zu können.

Das Messsignal US, IS das hier als erstes Messsignal M1 bezeichnet ist, wird nur dann an den Neutralanschluss 17 bzw. die Neutralelektrode 23 angelegt, wenn keine Behandlung des Gewebes 21 mittels der Arbeitselektrode 22 durchgeführt wird. Das erste Messsignal M1 wird somit außerhalb der Behandlungsperioden P angelegt. Dadurch ist sichergestellt, dass es keine gegenseitige Beeinflussung zwischen der Arbeitsspannung UA bzw. dem Arbeitsstrom IA einerseits und dem ersten Messsignal M1 oder einem daraus resultierenden Messstrom gibt.

In Figur 9 ist für das erste Messsignal M1 zu erkennen, dass in einem Zeitabschnitt zwischen zwei aufeinanderfolgenden Behandlungsperioden P die Messfrequenz ω konstant sein kann oder auch geändert werden kann. In dem dargestellten Beispiel sind im betrachteten Zeitraum vier unterschiedliche Messfrequenzen ω₁, ω₂, ω₃ und ω₄ des ersten Messsignals M1 dargestellt. Die Anzahl der verwendeten Messfrequenzen ω kann variieren und anwendungsabhängig gewählt werden. Die verwendeten Messfrequenzen werden aus einem Frequenzbereich von 10 Hz bis 1,0 MHz ausgewählt.

Die Amplitude des Wechselspannungsmesssignals US (Peak- oder Spitzenwert) ist insbesondere kleiner als 0,1 V oder kleiner als 50 mV. Beim Ausführungsbeispiel wird eine Amplitude von 10 mV verwendet. Die Amplitude eines Wechselstrommesssignals IS (Peak- oder Spitzenwert) ist insbesondere kleiner als 1,0 mA oder kleiner als 100 µA.

Die Zeitdauer oder die Anzahl der Perioden, während der das Messsignal US, IS mit einer unveränderten Messfrequenz ω anliegt, kann variieren. Diese Zeitdauer muss lediglich lang genug sein, um den daraus resultierenden Messstrom IM oder die daraus resultierende Messspannung UM zu erfassen, damit dann der Impedanzistwert Zᵢₛₜ aus dem angelegten Wechselspannungsmesssignal US und dem Messstrom IM gemäß dem ohmschen Gesetz ermittelt werden kann.

Zusätzlich oder alternativ kann das Messsignal US, IS zumindest in Zeitabschnitten während einer oder mehreren Behandlungsperioden P an den Neutralanschluss 18 bzw. die Neutralelektrode 23 angelegt werden, was in Figur 9 durch das als zweites Messsignal M2 bezeichnete Messsignal US, US dargestellt ist. In diesem Fall wird die Messfrequenz ω, die während einer Behandlungsperiode P verwendet wird, ausreichend unterschiedlich gewählt zur Arbeitsfrequenz der Arbeitsspannung UA und/oder des Arbeitsstromes IA. Durch diese Maßnahme lassen sich der Wechselspannungsmessstrom IS oder der resultierende Messstrom IM und der Arbeitsstrom IA unterscheiden, so dass die Ermittlung des Impedanzistwerts Zᵢₛₜ mit ausreichender Genauigkeit erfolgen kann.

Im Übrigen gilt für das zweite Messsignal M2 dasselbe wie für das erste Messsignal M1.

Zusätzlich oder alternativ zur Auswertung im Zeitbereich kann auch eine Auswertung im Frequenzbereich erfolgen. In Figur 10 ist stark schematisiert mit durchgezogenen Linien das Spektrum FA der Arbeitsstromes IA und mit gestrichelten Linien das Spektrum FM des Messtroms IM stark vereinfacht durch Spektrallinien dargestellt. Im Zeitbereich können die Arbeitsspannung UA und das Wechselspannungsmesssignal US und/oder der Arbeitsstrom IA und das Wechselstrommesssignal IS bzw. der Messstrom IM überlagert werden. Dabei kann der Signalverlauf des Messsignals US, IS im Zeitbereich derart gewählt werden, dass die Spektrallinien des Spektrums FM des Wechselstrommesssignals IS oder des Messstroms IM benachbart zu den Spektrallinien des Spektrums FA des Arbeitsstroms IA liegen, so dass eine Signaltrennung im Frequenzbereich möglich ist und somit die Impedanzistwerte im Wesentlichen unbeeinflusst von der Arbeitsspannung UA bzw. dem Arbeitsstrom IA ermittelt werden können.

Wie vorstehend erläutert, können die ermittelten Impedanzistwerte Zᵢₛₜ mit Hilfe von vorgegebenen Impedanzvergleichswerten geprüft werden. Zusätzlich oder alternativ können auch andere Prüfkriterien oder Prüfmethoden verwendet werden. Beispielsweise können Klassifikatoren auf die Impedanzistwerte angewandt werden, um daraus die Qualität der elektrischen Verbindung (beispielsweise den tatsächlich elektrisch leitend am Patienten anliegenden Anteil der verfügbaren Kontaktflächen 54) zu ermitteln. Hierzu können Klassifikatoren, wie beispielsweise der Bayes-Klassifikator, eine SVM (Support Vector Machine), ein künstliches neuronales Netz, ein Entscheidungsbaum oder andere mathematische/oder statistische Methoden zum Einsatz kommen.

Beispielsweise können einem künstlichen neuronalen Netz als Eingangsneuronen der Imaginärteil Im(Z) und der Realteil Re(Z) des Impedanzistwerts Zᵢₛₜ oder alternativ Betrag und Phase des Impedanzistwerts Zᵢₛₜ für jede definierte Messfrequenz ω innerhalb des gesamten Frequenzbereichs (z.B. von 10 Hz bis 1 MHz) übermittelt werden. Für jede definierte Messfrequenz ergeben sich dabei zwei Eingangsneuronen des neuronalen Netzes. Das künstliche neuronale Netz kann mittels Trainingsdaten trainiert werden und daraufhin nicht zulässige, mangelhafte elektrische Verbindungen zwischen der Neutralelektrode 23 und dem Patienten erkennen.

Die genannten Klassifikatoren können auch auf vorverarbeitete Größen bzw. Ersatzparameter angewandt werden, die beispielsweise aus einem oder mehreren gemessenen Impedanzistwerten Zᵢₛₜ und/oder den verfügbaren Parametern der Modellimpedanz 39 ermittelt werden. Beispielsweise ergeben sich durch die hier verwendete Modellimpedanz mehrere Variablen oder Parameter, die die Modellimpedanz 39 definieren. Beim Ausführungsbeispiel gemäß Figur 5 können dies fünf Parameter sein, nämlich die Beträge der Widerstände 43, 44, die Phasenlage des Konstantphasenelements 41, der Gleichstromwiderstand der Warburg-Impedanz 40 sowie die Zeitkonstante der Warburg-Impedanz 40. Durch eine optionale Dimensionsreduktion kann die Anzahl der Parameter reduziert werden, auf die die Klassifikatoren angewandt werden.

In Figur 8 ist lediglich beispielhaft dargestellt, dass Klassifikatoren auf zwei Ersatzparameter angewandt werden, nämlich eine erste Hauptkomponente HK1 und eine zweite Hauptkomponente HK2, die basierend auf den Parametern der Modellimpedanz 39 durch eine Hauptkomponentenanalyse (HKA) ermittelt wurden. Die Anzahl der verbleibenden Ersatzparameter ist kleiner als die Anzahl der Parameter der Modellimpedanz 39 und kann auch, anders als in Figur 8 dargestellt, größer oder kleiner als 2 sein.

In Figur 8 sind beispielhaft Felder dargestellt, die den prozentuellen Anteil der Kontaktflächen 54 angeben, der tatsächlich elektrisch leitend mit dem Patienten verbunden ist. Daraus lassen sich über die Anwendung von Klassifikatoren eine ausreichend elektrische Verbindung sowie eine unzureichend elektrische Verbindung zwischen der Neutralelektrode 23 und dem Patienten erkennen.

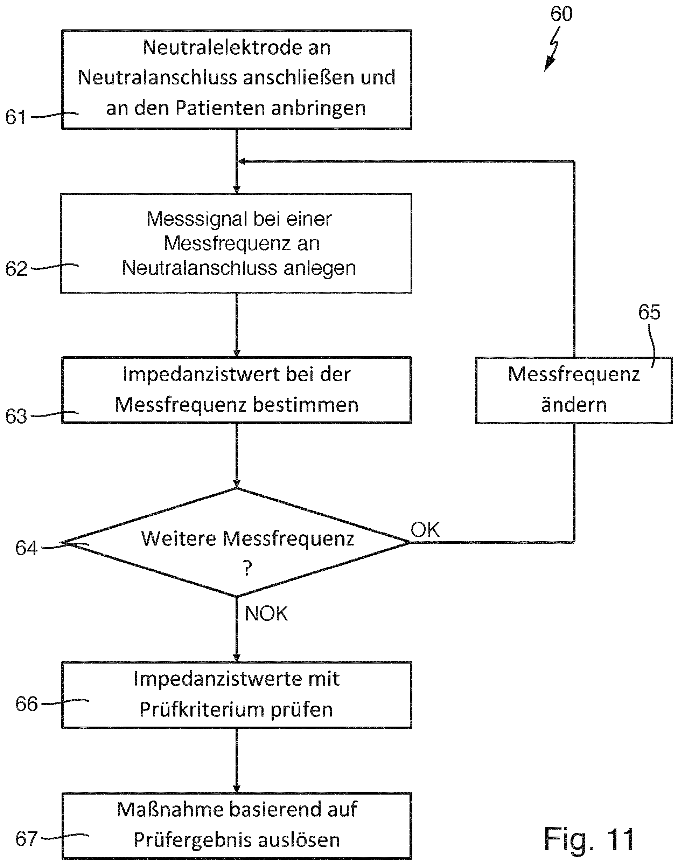

In Figur 11 ist ein Flussdiagramm eines Ausführungsbeispiels eines Verfahrens 60 veranschaulicht, mittels dem eine elektrische Verbindung zwischen einer Neutralelektrode 23 und einem Patienten geprüft werden kann.

In einem ersten Verfahrensschritt 61 wird die Neutralelektrode 23 an den Neutralanschluss 18 angeschlossen und am Patienten angebracht.

Im zweiten Verfahrensschritt 62 wird eine erste Messfrequenz ausgewählt und ein Messsignal US, IS bei der ausgewählten Messfrequenz an den Neutralanschluss 18 angelegt. Der daraus resultierende Messstrom IM oder die sich daraus ergebende Messspannung UM wird erfasst, so dass für die erste Messfrequenz ein erster Impedanzistwert Zᵢₛₜ ermittelt werden kann (dritter Schritt 63).

In einem vierten Verfahrensschritt 64 wird geprüft, ob die Impedanzwertermittlung für weitere Messfrequenzen ω erfolgen soll. Beispielsweise kann der Impedanzistwert bei mindestens 10 Messfrequenzen, mindestens 20 Messfrequenzen oder mehr Messfrequenzen ermittelt werden. Wenn noch nicht bei allen definierten Messfrequenzen eine Impedanzistwertermittlung durchgeführt wurde (Verzweigung OK aus dem vierten Verfahrensschritt 64), wird in einem fünften Verfahrensschritt 65 eine weitere Messfrequenz ausgewählt, die sich von der Messfrequenz oder den Messfrequenzen unterscheidet, bei denen der Impedanzistwert bisher ermittelt wurde. Im Anschluss an den fünften Verfahrensschritt 65 wird das Verfahren im zweiten Verfahrensschritt 62 fortgesetzt.

Andernfalls (Verzweigung NOK aus dem vierten Verfahrensschritt 64) wird das Verfahren in einem sechsten Verfahrensschritt 66 fortgesetzt.

Im sechsten Verfahrensschritt 66 werden die für die verschiedenen Messfrequenzen ω ermittelten Impedanzistwerte Zᵢₛₜ mit dem vorgegebenem Prüfkriterium verglichen. Das Prüfkriterium kann beispielsweise ein Vergleich mit mehreren vorgegebenen frequenzabhängigen Impedanzvergleichswerten beinhalten. Es kann zusätzlich oder alternativ die Anwendung von einem oder mehreren Klassifikatoren auf die ermittelten Impedanzistwerte und/oder daraus abgeleitete Parameter umfassen.

Das im sechsten Verfahrensschritt 66 ermittelte Prüfergebnis gibt an, ob die elektrisch leitende Verbindung zwischen der Neutralelektrode 23 und dem Patienten den Anforderungen entspricht oder unzureichend ist, so dass sich dadurch unzulässig hohe Stromdichten im Bereich der Neutralelektrode 23 innerhalb des Gewebes 21 ergeben können. Dies könnte wiederum zu endogenen Verbrennungen im Gewebe 21 führen.

In einem siebten Verfahrensschritt 67 wird eine vom Prüfergebnis abhängige Maßnahme eingeleitet. Zum Beispiel kann einer Bedienperson - beispielsweise über eine Schnittstelle am Versorgungsgerät 16 - eine Meldung ausgegeben werden, ob die elektrische Verbindung zwischen der Neutralelektrode 23 und dem Patienten den Anforderungen genügt. Die Meldung kann auch eine Information über die Qualität der elektrischen Verbindung beinhalten und beispielsweise charakteristisch sein für den Anteil der insgesamt verfügbaren Kontaktflächen 34, die elektrisch leitend mit dem Patienten verbunden sind.

Ist der elektrische Kontakt zwischen der Neutralelektrode 23 und dem Patienten derart mangelhaft, dass die Gefahr von Verbrennungen besteht, kann im siebten Verfahrensschritt 67 der Betrieb des Instruments 19 gestoppt werden, so dass kein weiterer Arbeitsstrom IA durch die Arbeitselektrode in das Gewebe 21 fließen kann. Beispielsweise kann das Erzeugen und Anlegen der Arbeitsspannung UA bzw. des Arbeitsstromes IA an den Versorgungsanschluss 17 unterbunden werden.

Die Erfindung betrifft ein Elektrochirurgiesystem 15 und ein Verfahren 60, das beim Betrieb des Elektrochirurgiesystems 15 angewandt werden kann. Das Elektrochirurgiesystem 15 hat ein Versorgungsgerät 16 sowie eine daran angeschlossene Neutralelektrode 23. An die Neutralelektrode 23 kann ein Wechselspannungsmesssignal US angelegt oder ein in die Neutralelektrode 23 fließendes Wechselstrommesssignal IS eingeprägt und der sich dabei ergebende Impedanzistwert Zᵢₛₜ des Neutralelektrodenstromkreises ermittelt werden. Das Messsignal (Wechselspannungsmesssignal US oder das Wechselstrommesssignal IS) wird bei mehreren verschiedenen Messfrequenzen ω angelegt bzw. eingeprägt und jeweils ein Impedanzistwert Zᵢₛₜ für die betreffende Messfrequenz ω ermittelt. Die daraus erhaltenen mehreren Impedanzistwerte kennzeichnen einen frequenzabhängigen Verlauf der Impedanz und können mit einem vorgegebenen frequenzabhängigen Prüfkriterium geprüft werden. Anhand der Prüfung wird erkannt, ob die elektrisch leitende Verbindung zwischen der Neutralelektrode 23 und dem Patienten den durch das Prüfkriterium definierten Vorgaben entspricht. Insbesondere wird geprüft, ob ein ausreichend großer Flächenanteil der Neutralelektrode 23 elektrisch leitend mit dem Patienten verbunden ist, so dass zu hohe Stromdichten im Bereich der Neutralelektrode 23 innerhalb des Gewebes 21 des Patienten vermieden werden können.

### Bezugszeichenliste:

- 15: Elektrochirurgiesystem
- 16: Versorgungsgerät
- 17: Versorgungsanschluss
- 18: Neutralanschluss
- 19: Instrument
- 20: Kabel für das Instruments
- 21: Gewebe
- 22: Arbeitselektrode
- 23: Neutralelektrode
- 24: Kabel für die Neutralelektrode
- 25: erster Leiter
- 26: zweiter Leiter

- 30: erster Elektrodenbereich
- 31: zweiter Elektrodenbereich
- 32: dritter Elektrodenbereich
- 33: Steg
- 34: Kontaktfläche
- 35: Anschlussbereich

- 39: Modellimpedanz
- 40: Warburg-Impedanz
- 41: Konstantphasenelement
- 42: Parallelschaltung
- 43: erster Widerstand
- 44: zweiter Widerstand
- 45: Messeinheit
- 46: Netzspannungsquelle

- 48: erste Impedanz
- 49: zweite Impedanz
- 50: Wechselrichterschaltung

- 60: Verfahren
- 61: erster Verfahrensschritt
- 62: zweiter Verfahrensschritt
- 63: dritter Verfahrensschritt
- 64: vierter Verfahrensschritt
- 65: fünfter Verfahrensschritt
- 66: sechster Verfahrensschritt
- 67: siebter Verfahrensschritt

- ω: Messfrequenz
- A_{w}: Warburgkoeffizient der Warburg-Impedanz
- B: Bezugsebene
- GND: Masse
- HK1: erste Hauptkomponente
- HK2: zweite Hauptkomponente
- IM: Messstrom
- IS: Wechselstrommesssignal
- L: Längsrichtung
- M1: erstes Messsignal
- M2: zweites Messsignal
- P: Behandlungsperiode
- Q: Querrichtung
- UA: Arbeitsspannung
- UM: Messspannung
- US: Wechselspannungsmesssignal
- Z: Impedanz
- Zᵢₛₜ: Impedanzistwert
- Zₘₐₓ: oberer Impedanzgrenzwert
- Zₘᵢₙ: unterer Impedanzgrenzwert

## Patentansprüche

1. Elektrochirurgiesystem (15) aufweisend ein Versorgungsgerät (16) mit einem Neutralanschluss (18) und eine an den Neutralanschluss (18) angeschlossene Neutralelektrode (23), die dazu eingerichtet ist, elektrisch leitend mit einem Patienten verbunden zu werden,
wobei das Versorgungsgerät (16) dazu eingerichtet ist, am Neutralanschluss (18) bei mehreren verschiedenen Messfrequenzen (ω) ein Messsignal (US, IS) bereitzustellen und für jede Messfrequenz (ω) einen Impedanzistwert (Zᵢₛₜ(ω)) der Neutralelektrode (23) zu ermitteln und die Impedanzistwerte (Zᵢₛₜ(ω)) anhand eines vorgegebenen frequenzabhängigen Prüfkriteriums zu prüfen,
**dadurch gekennzeichnet, dass** das Prüfkriterium auf einer frequenzabhängigen Modellimpedanz (39) basiert, die mehrere Parameter bereitstellt, um die Neutralelektrode (23) zu beschreiben einschließlich einer räumlichen Abhängigkeit der Flächenanteile der Kontaktfläche der Neutralelektrode (23), die eine ausreichend niederohmige Verbindung zum Patienten haben oder die keine ausreichend niederohmige elektrische Verbindung zum Patienten haben.

2. Elektrochirurgiesystem nach Anspruch 1, wobei die Neutralelektrode (23) einen elektrisch leitfähigen ersten Elektrodenbereich (30) und einen elektrisch leitfähigen zweiten Elektrodenbereich (31) aufweist, deren elektrischen Potentiale voneinander getrennt sind.

3. Elektrochirurgiesystem nach Anspruch 2, wobei der erste Elektrodenbereich (30) über einen ersten Leiter (25) und der zweite Elektrodenbereich (31) über einen zweiten Leiter (26) mit dem Neutralanschluss (18) elektrisch verbunden sind.

4. Elektrochirurgiesystem nach Anspruch 2 oder 3, wobei der erste Elektrodenbereich (30) und der zweite Elektrodenbereich (31) gleich große Flächeninhalte und/oder dieselbe Geometrie aufweisen.

5. Elektrochirurgiesystem nach einem der Ansprüche 2 bis 4, wobei der erste Elektrodenbereich (30) und der zweite Elektrodenbereich (31) mit Abstand zueinander und symmetrisch zu einer Bezugsebene (B) durch die Neutralelektrode (23) angeordnet sind.

6. Elektrochirurgiesystem nach einem der Ansprüche 2 bis 5, wobei die Neutralelektrode (23) einen elektrisch leitfähigen dritten Elektrodenbereich (32) aufweist, dessen elektrisches Potential getrennt ist von den elektrischen Potentialen des ersten Elektrodenbereichs (30) und des zweiten Elektrodenbereichs (31).

7. Elektrochirurgiesystem nach Anspruch 6, wobei der dritte Elektrodenbereich (32) den ersten Elektrodenbereich (30) und den zweiten Elektrodenbereich (31) umgibt.

8. Elektrochirurgiesystem nach einem der vorhergehenden Ansprüche, wobei das Prüfkriterium auf wenigstens einem vorgegebenen frequenzabhängigen Impedanzvergleichswert (Zₘₐₓ (ω) , Zₘᵢₙ (ω) ) basiert.

9. Elektrochirurgiesystem nach einem der vorhergehenden Ansprüche, wobei die Modellimpedanz (39) eine Warburg-Impedanz (40) aufweist.

10. Elektrochirurgiesystem nach Anspruch 9, wobei die Modellimpedanz (39) eine Parallelschaltung (42) aufweist, in der die Warburg-Impedanz (40) und ein Konstantphasenelement (41) parallel zueinander geschaltet sind.

11. Elektrochirurgiesystem nach einem der vorhergehenden Ansprüche, wobei das Versorgungsgerät (16) einem Versorgungsanschluss (17) und ein an den Versorgungsanschluss (17) elektrisch angeschlossenes Instrument (19) mit einer Arbeitselektrode (22) aufweist, wobei das Versorgungsgerät (16) und/oder das Instrument (19) dazu eingerichtet ist, der Arbeitselektrode (22) eine Arbeitsspannung (UA) und/oder einen Arbeitsstrom (IA) zuzuführen.

12. Elektrochirurgiesystem nach Anspruch 11, wobei das Versorgungsgerät (16) dazu eingerichtet ist, das Messsignal (US, IS) nur an den Neutralanschluss (18) anzulegen, wenn der Arbeitselektrode (22) keine Arbeitsspannung (UA) und kein Arbeitsstrom (IA) zugeführt wird.

13. Elektrochirurgiesystem nach Anspruch 11, wobei das Versorgungsgerät (16) dazu eingerichtet ist, die Messfrequenz (ω) des Messsignals (US, IS) verschieden einzustellen verglichen mit der Arbeitsfrequenz der Arbeitsspannung (UA) und/oder des Arbeitsstroms (IA), wenn das Messsignal (US, IS) an den Neutralanschluss (18) bereitgestellt wird, während der Arbeitselektrode (22) eine Arbeitsspannung (UA) und/oder ein Arbeitsstrom (IA) zugeführt wird.

14. Verfahren zum Prüfen der elektrischen Verbindung zwischen einer Neutralelektrode (23) und einem Patienten, wobei das Verfahren die folgenden Schritte umfasst:
- Anbringen der Neutralelektrode (23) am Patienten, so dass zwischen der Neutralelektrode (23) und dem Patienten eine elektrisch leitende Verbindung besteht,
- Anlegen jeweils eines Messsignals (US, IS) bei mehreren verschiedenen Messfrequenzen (ω) an die Neutralelektrode (23),
- Ermitteln einen Impedanzistwert (Zᵢₛₜ(ω)) für jede Messfrequenz (ω) des Messsignal (US, IS),
- Prüfen der Impedanzistwerte (Zᵢₛₜ(ω)) anhand eines vorgegebenen Prüfkriteriums, und
- Einleiten einer vom Prüfergebnis abhängigen Maßnahme,
**dadurch gekennzeichnet, dass** das Prüfkriterium auf einer frequenzabhängigen Modellimpedanz (39) basiert, die mehrere Parameter bereitstellt, um die Neutralelektrode (23) zu beschreiben einschließlich einer räumlichen Abhängigkeit der Flächenanteile der Kontaktfläche der Neutralelektrode (23), die eine ausreichend niederohmige Verbindung zum Patienten haben oder die keine ausreichend niederohmige elektrische Verbindung zum Patienten haben.

## Claims

1. An electrosurgical system (15) comprising a supply apparatus (16) having a neutral connection (18) and a neutral electrode (23) connected to the neutral connection (18), which is configured to be electrically conductively connected to a patient,
wherein the supply apparatus (16) is configured to provide a measurement signal (US, IS) at the neutral connection (18) at several different measurement frequencies (ω) and to determine an impedance actual value (Zᵢₛₜ(ω)) of the neutral electrode (23) for each measurement frequency (ω) and to check the impedance actual values (Zᵢₛₜ(ω)) based on a predefined frequency-dependent check criterion,
**characterized in that** the check criterion is based on a frequency-dependent model impedance (39) that provides several parameters to describe the neutral electrode (23), including a spatial dependence of the area fractions of the contact surface of the neutral electrode (23) that have a sufficiently low-resistance connection to the patient or that do not have a sufficiently low-resistance electrical connection to the patient.

2. The electrosurgical system according to claim 1, wherein the neutral electrode (23) comprises an electrically conductive first electrode section (30) and an electrically conductive second electrode section (31), the electrical potentials of which are separated from one another.

3. The electrosurgical system according to claim 2, wherein the first electrode section (30) is electrically connected to the neutral connection (18) via a first conductor (25) and the second electrode section (31) is electrically connected to the neutral connection (18) via a second conductor (26).

4. The electrosurgical system according to claim 2 or 3, wherein the first electrode section (30) and the second electrode section (31) comprise areas of equal size and/or identical geometries.

5. The electrosurgical system according to one of the claims 2 to 4, wherein the first electrode section (30) and the second electrode section (31) are arranged at a distance from each other and symmetrically to a reference plane (B) through the neutral electrode (23).

6. The electrosurgical system according to any of the claims 2 to 5, wherein the neutral electrode (23) comprises an electrically conductive third electrode section (32), the electrical potential of which is separated from the electrical potentials of the first electrode section (30) and the second electrode section (31).

7. The electrosurgical system according to claim 6, wherein the third electrode section (32) surrounds the first electrode section (30) and the second electrode section (31).

8. The electrosurgical system according to any of the preceding claims, wherein the check criterion is based on at least one predefined frequency-dependent impedance comparison value (Zₘₐₓ(ω), Zₘᵢₙ(ω)).

9. The electrosurgical system according to any of the preceding claims, wherein the model impedance (39) comprises a Warburg impedance (40).

10. The electrosurgical system according to claim 9, wherein the model impedance (39) comprises a parallel connection (42) in which the Warburg impedance (40) and a constant phase element (41) are connected in parallel to one another.

11. The electrosurgical system according to any of the preceding claims, wherein the supply apparatus (16) comprises a supply connection (17) and an instrument (19) electrically connected to the supply connection (17) and having an operating electrode (22), wherein the supply apparatus (16) and/or the instrument (19) is configured to supply an operating voltage (UA) and/or an operating current (IA) to the operating electrode (22).

12. The electrosurgical system according to claim 11,
wherein the supply apparatus (16) is configured to apply the measurement signal (US, IS) to the neutral connection (18) only when no operating voltage (UA) and no operating current (IA) are supplied to the operating electrode (22).

13. The electrosurgical system according to claim 11,
wherein the supply apparatus (16) is configured to set the measurement frequency (ω) of the measurement signal (US, IS) differently compared to the operating frequency of the operating voltage (UA) and/or the operating current (IA) when the measurement signal (US, IS) is provided to the neutral connection (18) while the operating electrode (22) is supplied with an operating voltage (UA) and/or an operating current (IA).

14. A method for checking the electrical connection between a neutral electrode (23) and a patient, wherein the method comprises the following steps:
- attaching the neutral electrode (23) to the patient so that an electrically conductive connection exists between the neutral electrode (23) and the patient,
- applying one measurement signal (US, IS) respectively at multiple different measurement frequencies (ω) to the neutral electrode (23),
- determining an impedance actual value (Zᵢₛₜ(ω)) for each measurement frequency (ω) of the measurement signal (US, IS),
- checking the impedance actual values (Zᵢₛₜ(ω)) based on a predefined check criterion, and
- initiating a measure depending on the check result,
- **characterized in that** the check criterion is based on a frequency-dependent model impedance (39) that provides several parameters to describe the neutral electrode (23), including a spatial dependence of the area fractions of the contact surface of the neutral electrode (23) that have a sufficiently low-resistance connection to the patient or that do not have a sufficiently low-resistance electrical connection to the patient.

## Revendications

1. Système d'électrochirurgie (15) présentant un appareil d'alimentation (16) doté d'une borne neutre (18) et d'une électrode neutre (23) qui est raccordée à la borne neutre (18) et est conçue pour être reliée de manière électriquement conductrice à un patient,
dans lequel l'appareil d'alimentation (16) est conçu pour fournir un signal de mesure (US, IS) sur la borne neutre (18), pour plusieurs fréquences de mesure (ω) différentes, et pour déterminer, pour chaque fréquence de mesure (ω), une valeur d'impédance réelle (Zᵢₛₜ(ω)) de l'électrode neutre (23) et vérifier les valeurs d'impédance réelles (Zᵢₛₜ(ω)) à l'aide d'un critère de vérification prédéfini dépendant de la fréquence,
**caractérisé en ce que** le critère de vérification est basé sur une impédance de modèle (39) dépendant de la fréquence, qui fournit plusieurs paramètres pour décrire l'électrode neutre (23), y compris une dépendance dans l'espace des parties de la surface de contact de l'électrode neutre (23) qui ont une liaison à valeur ohmique suffisamment faible avec le patient ou qui n'ont pas une liaison électrique à valeur ohmique suffisamment faible avec le patient.

2. Système d'électrochirurgie selon la revendication 1, dans lequel l'électrode neutre (23) présente une première zone d'électrode (30) conductrice de l'électricité et une deuxième zone d'électrode (31) conductrice de l'électricité, dont les potentiels sont séparés l'un de l'autre.

3. Système d'électrochirurgie selon la revendication 2, dans lequel la première zone d'électrode (30) est reliée électriquement à la borne neutre (18) par l'intermédiaire d'un premier conducteur (25) et la deuxième zone d'électrode (31) est reliée à la borne neutre par l'intermédiaire d'un deuxième conducteur (26).

4. Système d'électrochirurgie selon la revendication 2 ou 3, dans lequel la première zone d'électrode (30) et la deuxième zone d'électrode (31) présentent des superficies de mêmes dimensions et/ou ayant la même géométrie.

5. Système d'électrochirurgie selon une des revendications 2 à 4, dans lequel la première zone d'électrode (30) et la deuxième zone d'électrode (31) sont disposées à distance l'une de l'autre et de manière symétrique par rapport à un plan de référence (B) passant par l'électrode neutre (23).

6. Système d'électrochirurgie selon une des revendications 2 à 5, dans lequel l'électrode neutre (23) présente une troisième zone d'électrode (32) conductrice de l'électricité dont le potentiel électrique est séparé des potentiels électriques de la première zone d'électrode (30 et de la deuxième zone d'électrode (31).

7. Système d'électrochirurgie selon la revendication 6, dans lequel la troisième zone d'électrode (32) entoure la première zone d'électrode (30) et la deuxième zone d'électrode (31).

8. Système d'électrochirurgie selon une des revendications précédentes, dans lequel le critère de vérification est basé sur au moins une valeur de comparaison d'impédance (Zₘₐₓ(ω), Zₘᵢₙ(ω)) prédéfinie, dépendant de la fréquence.

9. Système d'électrochirurgie selon une des revendications précédentes, dans lequel l'impédance de modèle (39) présente une impédance de Warburg (40).

10. Système d'électrochirurgie selon la revendication 9, dans lequel l'impédance de modèle présente un montage en parallèle (42) dans lequel l'impédance de Warburg (40) et un élément à phase constante (41) sont montés en parallèle l'un avec l'autre.

11. Système d'électrochirurgie selon une des revendications précédentes, dans lequel l'appareil d'alimentation (16) présente une borne d'alimentation (17) et un instrument (19) raccordé électriquement à la borne d'alimentation (17) et doté d'une électrode de travail (22), l'appareil d'alimentation (16) et/ou l'instrument (19) étant conçus pour amener une tension de travail (UA) et/ou un courant de travail (IA) à l'électrode de travail (22).

12. S ystème d'électrochirurgie selon la revendication 11, dans lequel l'appareil d'alimentation (16) est conçu pour appliquer le signal de mesure (US, IS) uniquement à la borne neutre (18), lorsqu'aucune tension de travail (UA) et aucun courant de travail (IA) ne sont amenés à l'électrode de travail (22).

13. S ystème d'électrochirurgie selon la revendication 11, dans lequel l'appareil d'alimentation (16) est conçu pour régler la fréquence de mesure (ω) du signal de mesure (US, IS) différemment comparé à la fréquence de travail de la tension de travail (UA) et/ou du courant de travail (IA), lorsque le signal de mesure (US, IS) est mis à disposition pour la borne neutre (18) pendant qu'une tension de travail (UA) et/ou un courant de travail (IA) est amené(e) à l'électrode de travail (22).

14. Procédé de vérification de la connexion électrique entre une électrode neutre (23) et un patient, le procédé comprenant les étapes suivantes :
- mise en place de l'électrode neutre (23) sur le patient, de manière à ce qu'il existe une connexion conductrice de l'électricité entre l'électrode neutre (23) et le patient,
- application respectivement d'un signal de mesure (US, IS) à l'électrode neutre (23), pour plusieurs fréquences de mesure (ω) différentes,
- détermination d'une valeur d'impédance réelle (Zᵢₛₜ(ω)) pour chaque fréquence de mesure (ω) du signal de mesure (US, IS),
- vérification des valeurs d'impédance réelles (Zᵢₛₜ(ω)) à l'aide d'un critère de vérification prédéfini, et
- déclenchement d'une mesure dépendant du résultat de la vérification,
**caractérisé en ce que** le critère de vérification est basé sur une impédance de modèle (39) dépendant de la fréquence, qui fournit plusieurs paramètres pour décrire l'électrode neutre (23), y compris une dépendance dans l'espace des parties de la surface de contact de l'électrode neutre (23) qui ont une liaison à valeur ohmique suffisamment faible avec le patient ou qui n'ont pas une liaison électrique à valeur ohmique suffisamment faible avec le patient
